# EUROPEAN PATENT APPLICATION

(11) **EP 1 884 232 A1**
(43) Date of publication of application: **06.02.2008**
(21) Application number: 05741439.3
(22) Date of filing: 23.05.2005
(51) Int. Cl.: A61K 9/127, A61K 41/00, A61K 49/00, A61K 48/00, C12N 15/09, A61K 47/42

(54) **METHOD OF PRODUCING LIPOSOMES CONTAINING GAS ENCLOSED THEREIN**

(71) Applicant: Mebiopharm Co., Ltd., Tokyo 107-0052 (JP)
(72) Inventor: MARUYAMA, Kazuo, Kanagawa 2291101 (JP); TAKIZAWA, Tomoko, Kanagawa 2200202 (JP); HAGISAWA, Kosuke, Hokkaido 062-0932 (JP); NISHIOKA, Toshihiko, Saitama 331-0812 (JP); YANAGIE, Hironobu, Minato-ku Tokyo 106-0032 (JP)
(74) Representative: Hartz, Nikolai
(86) International application number: PCT/JP2005/009346
(87) International publication number: WO 2006/126244

(57) **Abstract**

Gas-filled liposomes that are useful for ultrasonography and ultrasound therapy are provided. A method of producing the gas-filled liposomes, including filling the void space in a sealed container containing a liposome suspension in a volume amounting to 20 to 80% of the inner capacity thereof with a fluoride gas or a nitrogen gas, and subjecting the container to an ultrasonication.

## Description

### Technical Field

The present invention relates to gas-filled liposomes that are useful as an agent for ultrasonography and an agent for ultrasound therapy and relates to a method of producing the same.

### Background Art

Ultrasound diagnostic apparatuses are utilized in various fields, such as obstetrics and gynecology, cardiology, and urology. In addition, a diagnostic method has been developed, in which microparticles containing a gas (microbubbles) are administered as an ultrasound imaging agent and then imaging by ultrasonication (refer to Patent Documents 1 to 5).

However, conventional microbubbles are macroparticles made of albumin containing a gas and accordingly have a large particle diameter of 2 to 6 µm. Consequently, the penetration of the microbubbles into deep tissues is poor.
[Patent Document 1] US Patent No. 4572205
[Patent Document 2] US Patent No. 4718433
[Patent Document 3] US Patent No. 4774958
[Patent Document 4] US Patent No. 4844852
[Patent Document 5] US Patent No. 4957656

### Disclosure of the Invention

### Problems to be Solved by the Invention

Accordingly, it is an object of the present invention to provide a method of producing stable microbubbles having a small particle diameter by a simple process, and the microbubbles thus obtained and a method using thereof.

### Means for Solving the Problems

The present inventors have conducted various studies and have found that gas-filled liposomes having a small particle diameter can be stably prepared by preparing a suspension of previously produced liposomes, adding the suspension to a sealed container with a predetermined void space, filling the void space with a fluoride gas or a nitrogen gas, and then subjecting the container to an ultrasonication. In addition, the present inventors have found that the gas-filled liposomes having a small particle diameter can be stably obtained since the particle diameter of the liposomes prepared as the above depends on the particle diameter of the previously produced liposomes, and that the gas-filled liposomes are therefore useful as an agent for ultrasonography. Furthermore, the gas-filled liposomes readily explode by exposing them to a low-frequency ultrasonication, and thus they can be exploded selectively at target region when the gas-filled liposome having a ligand to a target cell or the like are prepared from a liposome to which the ligand is bound. Then, the present inventors have found that the gas-filled liposomes are also useful as an ultrasound therapeutic agent or a gene delivery composition.

Accordingly, the present invention provides a method of producing gas-filled liposomes, including filling the void space of a sealed container containing a suspension of liposomes in a volume amounting to 20 to 80% of the inner capacity thereof with a fluoride gas or a nitrogen gas, and subjecting the container to ultrasonication. The present invention also provides a gas-filled liposome thus obtained.

Furthermore, the present invention provides an agent for ultrasonography containing the gas-filled liposomes.
Furthermore, the present invention provides an ultrasound therapeutic agent containing the gas-filled liposomes, wherein the agent is administered and then a target region is exposed to a low-frequency ultrasonication.
Furthermore, the present invention provides a composition for delivering a gene into a cell with a low-frequency ultrasonication, containing the gas-filled liposomes and a gene or the like.
Furthermore, the present invention provides a method of delivering a gene or the like to a cultured cell, including adding the gas-filled liposomes and the gene or the like to the cultured cell, and exposing them to a low-frequency ultrasonication.
Furthermore, the present invention provides an ultrasound therapeutic agent containing the gas-filled liposomes and a medicinal component, wherein the agent is administered and then a target region is exposed to a low-frequency ultrasonication.

In addition, the present invention provides a use of the gas-filled liposomes for producing an agent for ultrasonography.
Furthermore, the present invention provides a use of the gas-filled liposomes for producing an ultrasound therapeutic agent, wherein the agent is administered and then a target region is exposed to a low-frequency ultrasonication.
Furthermore, the present invention provides a use of a composition containing the gas-filled liposomes and a medicinal component for producing an ultrasound therapeutic agent, wherein the agent is administered and then a target region is exposed to a low-frequency ultrasonication.

In addition, the present invention provides an ultrasonography, including administering the gas-filled liposomes and conducting ultrasonication.
Furthermore, the present invention provides an ultrasound therapy, including containing the gas-filled liposomes and exposing a target region to a low-frequency ultrasonication.
Furthermore, the present invention provides an ultrasound therapy, including administering the gas-filled liposome and a medicinal component and exposing a target region to a low-frequency ultrasonication.

### Effect of the Invention

According to the present invention, the gas-filled liposomes having a small and uniform particle diameter can be simply and stably prepared since the particle diameter thereof depends on raw liposomes. In addition, since the gas-filled liposomes have a small particle diameter, a large amount of the gas-filled liposomes can be delivered to a thrombus or an arteriosclerosis lesion formed in the arteriole or the like. Hence, the present invention can achieve to image a lesion that cannot be conventionally diagnosed and to treat a thrombus or an arteriosclerosis lesion in small blood vessels.
Furthermore, the exposure of a target region to a low-frequency ultrasound after administration of the gas-filled liposomes of the present invention causes cavitation due to microbubbles of the gas enclosed in the liposomes at the target region. Thus, the gas-filled liposomes have explosive property. Accordingly, a thrombus, an arteriosclerosis lesion or the like is treated by destructing thereof. In addition, the therapy can be conducted in more site-specific manner by using the liposomes to which a ligand to the lesion is bound. Furthermore, a gene can be delivered to a target cell with extremely high efficiency by simultaneously applying the gas-filled liposomes and the gene to the target cell in vivo or in vitro, and conducting low-frequency ultrasound exposure to generate microbubbles of the gas and to cause cavitation.

### Best Mode for Carrying Out the Invention

According to the present invention, liposomes are previously prepared. The liposomes used in the present invention are liposomes basically containing a lipid as a membrane constituent. The liposomes may include a drug, a gene, or the like in the inside thereof. Examples of the lipid used as the membrane constituent of the liposomes include phospholipids, glyceroglycolipids and sphingoglycolipids; cationic lipids in which a primary amino group, a secondary amino group, a tertiary amino group or a quaternary ammonium group is introduced into the above lipids; lipids in which polyalkylene glycols are introduced into the above lipids; and lipids to which a ligand to various types of cells, tissues and the like is bound.

The phospholipids are natural or synthetic phospholipids, such as phosphatidylcholines (e.g., soybean phosphatidylcholine, egg yolk phosphatidylcholine, distearoyl phosphatidylcholine, and dipalmitoyl phosphatidylcholine), phosphatidylethanolamines (e.g., distearoyl phosphatidylethanolamine), phosphatidylserines, phosphatidic acid, phosphatidylglycerols, phosphatidylinositols, lysophosphatidylcholines, sphingomyelins, egg yolk lecithins, soybean lecithins, and hydrogen added phospholipids.

Examples of the glyceroglycolipids include sulfoxyribosyl glycerides, diglycosyl diglycerides, digalactosyl diglycerides, galactosyl diglycerides, and glycosyl diglycerides. Examples of the sphingoglycolipids include galactosyl cerebrosides, lactosyl cerebrosides, and gangliosides.

Examples of the cationic lipids include lipids in which a quaternary ammonium group, such as an amino group, an alkylamino group, a dialkylamino group, a trialkylammonium group, a monoacyloxyalkyldialkylammonium group, or a diacyloxyalkylmonoalkylammonium group, is introduced into the above phospholipids, glyceroglycolipids, or sphingoglycolipids. Examples of the polyalkylene glycol-modified lipids include lipids in which the above phospholipids, glyceroglycolipids, or sphingoglycolipids are modified with polyethylene glycol, polypropylene glycol, or the like, such as di-C₁₂₋₂₄acyl-glycerolphosphatidylethanolamine-N-PEG.

In addition, cholesterols, and tocopherols, stearylamines, dicetylphosphates or gangliosides may optionally be used as a membrane stabilizer and an antioxidant, respectively.

Examples of the ligand that binds to a target cell, a target tissue or a target lesion include ligands that bind to cancer cells, such as transferrin, folic acid, hyaluronic acid, galactose and mannose; and ligands that bind to thrombi, such as RGD peptide and sigma protein. In addition, monoclonal antibodies and polyclonal antibodies can be used as the ligands.

The previously prepared liposomes have an aqueous phase in their inside, but may not necessarily include a medicinal component, a gene or the like. However, when used as the aforementioned therapeutic agent or the gene delivery composition, the liposomes may include a medicinal component, a gene or the like. Such a medicinal component includes, for example, a cancer therapeutic agent such as doxorubicin, 5-FU, a platinum derivative such as cisplatin or oxaliplatin, taxol, or camptothecine; a thrombolytic agent such as t-PA, urokinase, or streptokinase; an arteriosclerosis-treating agent such as prostaglandin; and an NF-kappa B or decoy against arterial occlusion or Buerger's disease. Examples of the gene include DNAs, RNAs, antisense DNAs, siRNAs, decoys, and therapeutic oligonucleotides.

The liposomes can be produced by a known process for preparing liposomes, for example, by the liposome preparation method of Bangham et al., (J. Mol. Biol., (1965), 13, 238), an ethanol injection method (J. Cell. Biol., (1975), 66, 621), a French press method (FEBS Lett., (1979), 99, 210), a freeze and thawing method (Arch. Biochem. Biophys., (1981), 212, 186), and a reverse phase evaporation method (Proc. Natl. Acad. Sci. USA, (1978), 75, 4194). For example, a liposome suspension is prepared by dissolving a lipid in an organic solvent, adding an aqueous solution thereto, and then treating the resulting mixture with an ultrasound. Then, if necessary, the suspension is applied to an extruder and/or a membrane filter for particle sizing. In such a case, the particles are preferably sized to have a particle diameter of 1 µm or less, more preferably 100 to 800 nm, and particularly preferably 100 to 600 nm.

The prepared liposome suspension is poured in a sealed container. In this stage, the void space of the container is preferably 20 to 80%, more preferably 30 to 80%, and particularly preferably 50 to 80% of the inner capacity of the container. When the void space is less than 20%, the introduction ratio of a gas to the produced liposomes is too low. The space exceeding 80% is uneconomical.

This void space is filled with a fluoride gas or a nitrogen gas. Examples of the fluoride gas include sulfur hexafluoride and perfluorohydrocarbon gases, such as CF₄, C₂F₆, C₃F₈, C₄F₁₀, C₅F₁₂, and C₆F₁₄. Among them, C₃F₈, C₄F₁₀, and C₅F₁₂ are particularly preferred. A nitrogen gas can also be used. The pressure of the filled gas is preferably 1 atm (gauge pressure) or more and particularly preferably 1 to 1.5 atms. A simple way for filling the void space with a gas is injection, for example, with a needle syringe though a rubber stopper. An injection cylinder may also be used.

Subsequently, an ultrasound treatment is conducted. For example, the container may be exposed to an ultrasound of 20 to 50 kHz for 1 to 5 minutes. With this ultrasound treatment, the aqueous solution in the liposomes is replaced with a fluoride gas or a nitrogen gas to give gas-filled liposomes. The given gas-filled liposomes have a particle diameter approximately the same as that of the raw liposomes. Accordingly, the gas-filled liposomes having a particle diameter within a certain range, e.g., 1 µm or less, more preferably 50 to 800 nm, and particularly preferably 100 to 600 nm, can be readily produced by sizing the raw liposomes when they are prepared.

Furthermore, the gas-filled liposomes can be readily produced at a site, such as a hospital, only by an ultrasound treatment, if a sealed container containing a liposome suspension and filled with a fluoride gas or a nitrogen gas is previously prepared and supplied to the hospital.

The gas-filled liposomes thus obtained can have a small particle diameter and a constant particle size distribution, and can be delivered to a microvasculature, a deep tissue, or the like. Accordingly, the imaging of a microvasculature, a deep tissue or the like that is too small or too deep to diagnose for conventional ultrasound imaging with microbubbles can be achieved by using the above gas-filled liposomes. Furthermore, the image becomes finer compared to the conventional methods. The ultrasonography using the gas-filled liposomes according to the present invention may be conducted by a conventional procedure. For example, an image of a tissue can be obtained by administering the gas-filled liposomes of the present invention and by exposing the tissue to a diagnostic ultrasound (2 to 6 MHz). The gas-filled liposomes are intravenously administered, for example.

When the gas-filled liposomes of the present invention are exposed to a low-frequency ultrasound containing a resonance frequency of 0.5 to 2 MHz, cavitation is caused by the disruption of the liposomes and microbubbles of the gas. If the cavitation is caused at a thrombus region, the thrombus is broken. In addition, if the liposomes include a ligand to a target lesion such as a thrombus or an arteriosclerosis lesion, the administered gas-filled liposomes of the present invention bind to the thrombus or the arteriosclerosis lesion. The binding can be traced by exposure to the diagnostic ultrasound. The thrombus or the arteriosclerosis lesion can be treated by exposing the lesions to a low-frequency ultrasound when the gas-filled liposomes have been bound to the region and exploding the liposomes to disrupt the lesions. Examples of disorders that can be treated with the explosion of the gas-filled liposomes include thrombi, arterioscleroses, angiitides, and cancerous tissues.

Furthermore, as described above, the gas-filled liposomes of the present invention can contain various medicinal components or genes. Accordingly, the medicinal component or the gene contained in the gas-filled liposomes can be delivered to target cells by administering the gas-filled liposomes, tracing the liposomes using a diagnostic ultrasound until they reach a target region, and exposing the target region to a low-frequency ultrasound once the liposomes have reached there to release the medicinal components or genes from the liposomes through the cavitation caused by microbubbles of the gas enclosed in the liposomes. Alternatively, the medicinal component or the gene may not be enclosed in the gas-filled liposomes of the present invention, but may be simultaneously administered with the gas-filled liposomes. The liposomes used for delivering genes are preferably liposomes prepared by using a cationic lipid. In addition, the delivery efficiency of the gene can be further improved by simultaneously administering protamine, polylysine, or the like.

The delivery efficiency of the gene to target cells is improved by the explosion of the gas-filled liposomes. Hence, a gene can be efficiently delivered to target cells by exposing the target region to a low-frequency ultrasound after administration of the gas-filled liposomes of the present invention and the gene. Furthermore, a gene can be delivered to cells in vitro, namely, to cultured cells. In such a case, the gas-filled liposomes of the present invention and a gene may be added to cultured cells and then a low-frequency ultrasound is exposed thereto.

Furthermore, in order to efficiently deliver a medicinal component or a gene to a target cell, a target tissue, a target lesion or the like, liposomes to which a ligand to such a target is bound are preferably used as the gas-filled liposomes according to the present invention.

### Examples

The present invention will hereinafter be described in detail with reference to the example, but is not limited thereto.
The followings are abbreviations used in Examples:
DPPC: dipalmitoyl phosphatidylcholine
DPPE: dipalmitoyl phosphatidylethanolamine
PEG: polyethylene glycol
Mal: maltose
DC-Chol: 3β-[N-(N',N'-dimethylaminoethane]carbamoyl]cholesterol
DOPE: dioleoyl phosphatidylethanolamine
DOTAP: 1,2-dioleoyl-3-trimethylammonium-propane
DPTMA: N-[1-(2,3-dioleoyloxy)propyl]-N,N,N-trimethylammonium chloride
DDAB: didodecyldimethylammonium bromide

### Example 1

### (1) Preparation of liposomes for targeting thrombus

Lipids of DPPC : cholesterol : DPPE-PEG : DPPE-PEG-Mal (1:1:0.11:0.02, (m/m)) were dissolved in an organic solvent mixture of chloroform : isopropyl ether (1:1, v/v), and an aqueous solution such as saline (or an aqueous solution containing a drug) was added thereto in a volume amounting to a half of the organic solvent mixture (at this stage, chloroform: isopropyl ether: aqueous solution = 1:1:1, v/v). The resulting mixture was mixed to give an emulsion. This emulsion was used for preparing liposomes by a reverse phase evaporation method (REV method). The liposomes were sized by passing them through polycarbonate menbranes of 400 nm, 200 nm and 100 nm with an extruder. PEG-liposomes can be maintained in a dispersion state for a long time (two years). However, the lipid composition does not influence the gas enclosure step, because any liposomes can be obtained in a dispersion state by enclosing the gas using an ultrasound.
The CGGGRGDF peptide was added to this liposome solution and reacted therewith at room temperature for 1 hour. Then, the mixture was ultracentrifugated to yield RGD-modified PEG-liposomes.

### (2) Preparation of a cationic liposome for gene delivery

### i) DC-Chol liposome

DOPE : DC-Chol = 2:3 (m/m) were dissolved in chloroform, and the mixture was put in a pear-shaped flask. The organic solvent was evaporated while rotating with a rotary evaporator to produce a thin film of a lipid on the wall (production of a lipid film). Then, liposomes were produced by hydration using a solvent such as saline. The liposomes were reduced in size by an ultrasound treatment or sized by passing them through polycarbonate membranes of 400 nm, 200 nm and 100 nm with an extruder.

### ii) DOTAP liposome

DOTAP: DOPE = 1:1 (w/w) were dissolved in chloroform, and the mixture was put in a pear-shaped flask. The organic solvent was evaporated while rotating with a rotary evaporator to produce a thin film of a lipid on the wall (production of a lipid film). Then, liposomes were produced by hydration using a solvent such as saline. The liposomes were reduced in size by an ultrasound treatment or sized by passing them through polycarbonate membranes of 400 nm, 200 nm and 100 nm with an extruder.

The following reagents were used as commercially available gene-delivering reagents composed of cationic liposomes:
Lipofectin^{™} (DOTMA : DOPE = 1:1, w/w), and
LipofectACE^{™} (DDAB : DOPE = 1:1.25, w/w).

### (3) Method of enclosing gas in various types of liposomes

A liposome aqueous solution (lipid concentration: 5 mg/mL) was put in a vial (5 mL, 10 mL, or 20 mL, for example) in a volume amounting to 30% of the capacity thereof (1.5 mL, 3 mL, or 6 mL), and perfluoropropane was put therein so that the air was replaced with the perfluoropropane. The vial was sealed with a rubber stopper, and perfluoropropane was further added through the rubber stopper with a needle syringe, to the volume amounting of 1.5 times of the inner capacity, so that the inner pressure became about 1.5 atms. A bath-type ultrasound apparatus (42 kHz) was filled with water, and the vial was left standing therein and exposed to an ultrasound for one minute.

### (4) Imaging of thrombus

A thrombus was artificially induced in a rabbit iliac artery by inserting a balloon catheter therein to scrape the endothelium. RGD-PEG-liposomes were intravenously injected to the rabbit. A diagnostic ultrasound apparatus (3.5 MHz) was used to detect the thrombus region, and the thrombus region was detected as the region showing increased brightness.

### (5) Disruption of thrombus

A thrombus was formed in a test tube, and the gas-filled RGD-PEG-liposomes prepared in the above (3) were added into the test tube. The mixture was left standing for 30 minutes, followed by removing the liposome solution. When the mixture was exposed to 1 MHz of a therapeutic ultrasound with saline, disruption of the surface was observed. While the same procedure was conducted with gas-filled PEG-liposomes, disruption was not observed. This difference was caused by binding of the gas-filled liposomes to the thrombus via the RGD peptide, indicating that targeting was achieved.

(6) Human pancreatic carcinoma cell line, AsPC-1, (4 x 10⁴ cells/well) was cultured in a 48-well plate, and an FITC-labelled oligonucleotide (18 nucleic acid residues) and the gas-filled PEG-liposomes prepared in the above (3) were added thereto. After exposure to a pulsed ultrasound of 1 MHz for three seconds, the culture solution was immediately washed three or four times repeatedly. Then, the fluorescent intensity in the cells was observed by a fluorescence microscope. The fluorescence was observed in the cells exposed to a low-frequency ultrasound. This result proves that a gene of interest can be delivered to target cells by adding the gas-filled liposomes and the gene to the cells and then exposing the mixture to a low-frequency ultrasound.

(7) A plasmid DNA coding luciferase and protamine were mixed to prepare a DNA-protamine complex for reducing the size. AsPC-1 cells (4 x 10⁴ cells/well) were cultured in a 48-well plate, and the DNA-protamine complex (1 µg of DNA, lipid : DNA = 12:1, w/w) and the gas-filled PEG-liposomes were added thereto. After exposure to a pulsed ultrasound of 1 MHz for three seconds, the culture solution was immediately washed three or four times repeatedly. After addition of a culture medium, the cells were further cultured for two days. Then, luciferase activity was measured by a conventional method. Table 1 shows the results.

**Table 1**

| Liposome | Treatment with ultrasound | Luciferase expression (RLU/mg protein) |
|---|---|---|
| DPPC LP | | ND |
| DPPC LP | +SONIC | 8×10⁶ |
| DC-Chol LP | | ND |
| DC-Chol LP | +SONIC | 66×10⁶ |
| DOTAP LP | | ND |
| DOTAP LP | +SONIC | 140×10⁶ |
| Lipofectin^{™} | | 1×10² |
| Lipofectin^{™} | +SONIC | 80×10⁶ |
| LipofectACE^{™} | | ND |
| LipofectACE^{™} | +SONIC | 78×10⁶ |

| | | |
|---|---|---|
| ND: not detected | | |

As shown in Table 1, it was confirmed that a gene could be efficiently delivered to cells by adding gas-filled liposomes and a gene to the cells and then exposing them to a low-frequency ultrasonication.

## Claims

1. A method of producing gas-filled liposomes, comprising filling the void space of a sealed container containing a suspension of liposomes in a volume amounting to 20 to 80% of the inner capacity thereof with a fluoride gas or a nitrogen gas, and subjecting the container to an ultrasonication.

2. The method according to Claim 1, wherein the liposomes are cationic lipid-containing liposomes.

3. The method according to Claim 1 or 2, wherein the liposomes include a ligand to a target cell, a target tissue, or a target lesion.

4. A gas-filled liposome prepared by the method according to any one of Claims 1 to 3.

5. An agent for ultrasonography, comprising the gas-filled liposomes according to Claim 4.

6. An ultrasound therapeutic agent comprising the gas-filled liposomes according to Claim 4, wherein the agent is administered and then a target region is exposed to a low-frequency ultrasonication.

7. A composition for delivering a gene into a cell with a low-frequency ultrasonication, comprising the gas-filled liposomes according to Claim 4 and a gene or the like.

8. A method of delivering a gene or the like to a cultured cell, comprising adding the gas-filled liposomes according to Claim 4 and the gene or the like to the cultured cell and exposing them to a low-frequency ultrasonication.

9. An ultrasound therapeutic agent, comprising the gas-filled liposomes according to Claim 4 and a medicinal component, wherein the agent is administered and then a target region is exposed to a low-frequency ultrasonication.

10. A use of the gas-filled liposomes according to Claim 4 for producing an agent for ultrasonography.

11. A use of the gas-filled liposomes according to Claim 4 for producing an ultrasound therapeutic agent, wherein the agent is administered and then a target region is exposed to a low-frequency ultrasonication.

12. A use of a composition comprising the gas-filled liposomes according to Claim 4 and a medicinal component for producing an ultrasound therapeutic agent, wherein the agent is administered and then a target region is exposed to a low-frequency ultrasonication.

13. An ultrasonography comprising administering the gas-filled liposomes according to Claim 4 and conducting ultrasonication.

14. An ultrasound therapy comprising administering the gas-filled liposomes according to Claim 4 and exposing a target region to a low-frequency ultrasonication.

15. An ultrasound therapy comprising administering the gas-filled liposomes according Claim 4 and a medicinal component, and exposing a target region to a low-frequency ultrasonication.
